# EUROPEAN PATENT APPLICATION

(11) **EP 4 790 391 A1**
(43) Date of publication of application: **12.08.2026**
(21) Application number: 26155971.0
(22) Date of filing: 03.02.2026
(51) Int. Cl.: G01N 21/75, G01N 21/77, G01N 21/552, G01N 33/543

(54) **OPTICAL MEASUREMENT METHOD, OPTICAL MEASUREMENT SYSTEM, AND TEST KIT**

(30) Priority: 06.02.2025 JP 2025018565
(71) Applicant: Canon Kabushiki Kaisha, Tokyo, 146-8501 (JP)
(72) Inventor: TAKAHASHI, Atsushi, Tokyo (JP); SASAGURI, Daisuke, Tokyo (JP); ABE, Shigemoto, Tokyo (JP)
(74) Representative: WESER & Kollegen Patentanwälte PartmbB

(57) **Abstract**

Provided is an optical measurement method capable of achieving determination of presence of a target substance using particles in a short time. The optical measurement method includes: a preparation step of preparing an optical measurement device (110), a reaction tank (112) in the optical measurement device (110) accommodating a sample containing magnetic particles on each of which a substance that specifically binds to a target substance is immobilized; a magnetic field application step of applying a magnetic field to the magnetic particles by a magnetic field application unit (113); an inspection value acquisition step of acquiring an inspection value based on an amount of light detected by a light detection unit (114) under a state in which the magnetic field is applied; and a determination step of performing determination of one of presence or absence of the target substance in the sample based on the inspection value.

## Description

### TECHNICAL FIELD

The embodiment disclosed in this specification and the drawings relates to an optical measurement method, an optical measurement system, and a test kit.

### BACKGROUND

In the field of biosensors, there is an optical measurement method in which an optical measurement system using a substrate having optical transparency optically measures a target substance such as an antigen contained in a specimen and determines whether or not the target substance is present in the specimen. The optical measurement method utilizes a change in a detection signal that occurs when carrier particles on which a first substance that specifically binds to the target substance is immobilized settle on a detection surface of the transmissive substrate.

In general, in detection of a target substance using particles, there is a method of separating particles bound to the target substance from particles not bound to the target substance.

In Japanese Patent Laid-Open No. 2018-40795, there is described a method in which, in the above-mentioned optical measurement method, magnetic particles are settled on a detection surface on which a second substance that specifically binds to the target substance is immobilized, and then a magnetic field is applied in a direction away from the detection surface. In the method as described in Japanese Patent Laid-Open No. 2018-40795, magnetic particles that have captured the target substance bind to the second substance on the detection surface through the target substance, to thereby remain on the detection surface even after the magnetic field is applied in the direction away from the detection surface. Meanwhile, magnetic particles that have not captured the target substance move away from the detection surface along the magnetic field, and are thus separated from the magnetic particles that have captured the target substance, thereby enabling detection of the target substance using the particles.

### SUMMARY

Currently, there is a demand for development of a measurement method for determining presence of a target substance in a short time, and the technology as described in Japanese Patent Laid-Open No. 2018-40795 has room for improvement in terms of shortening a measurement time.

That is, the present disclosure is directed to providing an optical measurement method capable of achieving determination of presence of a target substance using particles in a short time.

The present disclosure in its first aspect provides an optical measurement method as specified in claim 1. Optional features are specified in claims 2 to 14.

The present disclosure in its second aspect provides an optical measurement system as specified in claim 15. Optional features are specified in claim 16.

The present disclosure in its third aspect provides a test kit for use in the above-mentioned optical measurement method as specified in claim 17.

According to the present disclosure, the optical measurement method capable of achieving determination of presence of a target substance using particles in a short time can be provided.

Features of the present disclosure will become apparent from the following description of embodiments with reference to the attached drawings. The following description of embodiments is described by way of example.

### BRIEF DESCRIPTION OF THE DRAWINGS

Fig. 1 is a functional block diagram for illustrating a configuration example of an optical measurement system according to an embodiment.
Fig. 2 is a schematic diagram for illustrating a configuration example of the optical measurement system according to the embodiment.
Fig. 3 is a cross-sectional view for illustrating a structure example of a magnetic particle.
Fig. 4 is a schematic view for illustrating agglomeration behavior of magnetic particles.
Fig. 5 is a flow chart for illustrating a flow of an optical measurement method according to the embodiment.
Fig. 6A is a schematic view for illustrating behavior of magnetic particles exhibited when a magnetic field is applied to a positive specimen containing target substances.
Fig. 6B is a schematic view for illustrating behavior of magnetic particles exhibited when a magnetic field is applied to a negative specimen not containing the target substances.
Fig. 7 is a graph for showing an example of temporal changes in a signal intensity.

### DESCRIPTION OF THE EMBODIMENTS

An embodiment of an optical measurement method, an optical measurement system, and a test kit according to the present disclosure is described in detail below with reference to the drawings.

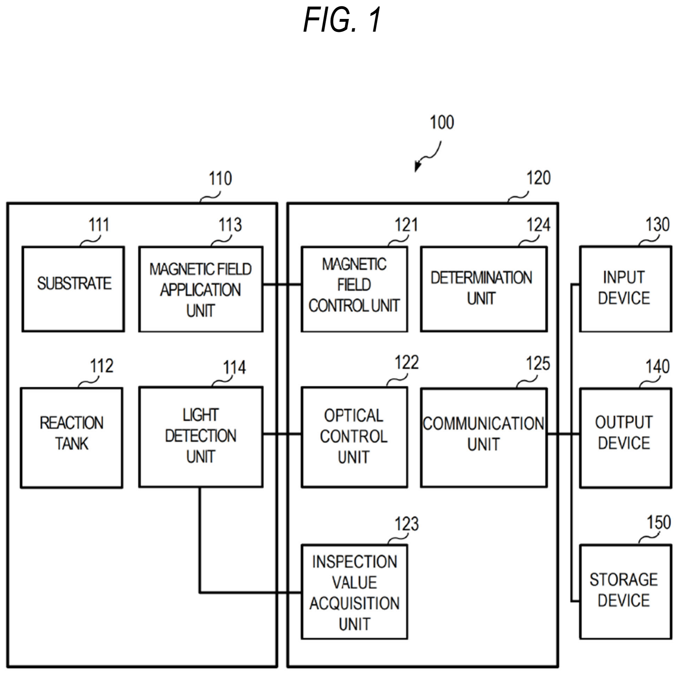

Fig. 1 is a functional block diagram for illustrating a configuration example of an optical measurement system 100 according to one embodiment of the present disclosure.

The optical measurement system 100 is a system that optically measures a target substance.

The target substance is not particularly limited as long as the target substance is a substance that can be detected by the optical measurement system 100. Examples thereof include antigens of an influenza virus, an adenovirus, a respiratory syncytial (RS) virus, and a coronavirus (such as COVID-19).

The optical measurement system 100 illustrated in Fig. 1 includes an optical measurement device 110, a signal processing device 120, an input device 130, an output device 140, and a storage device 150.

The optical measurement device 110 performs optical measurement under control of the signal processing device 120, and results of the measurement are transmitted to the signal processing device 120 to be subjected to processing for determining presence or absence of a target substance. The input device 130 is a device for an operator to input an instruction to operate the signal processing device 120, and the output device 140 is a device for outputting, to the operator, the results processed by the signal processing device 120. The storage device 150 is a device for storing pieces of information such as measurement data.

As the input device 130, for example, a physical switch, a touch panel, a touch pad, a joystick, a keyboard, a mouse, or the like can be used. As the input device 130, a voice input device that recognizes speech uttered by the operator sensed by a microphone and converts the speech into an operation signal may also be used.

As the output device 140, for example, a liquid crystal display (LCD), a cathode ray tube (CRT) display, an organic electro luminescence display (organic EL display; OELD), a plasma display, or any other display can be used as appropriate. The output device 140 may include a projector. The output device 140 may also include an apparatus that produces sound, such as a speaker.

The storage device 150 is a device that stores various types of information. Examples of the storage device 150 may include a read only memory (ROM), a random access memory (RAM), a hard disk drive (HDD), a solid state drive (SSD), and an integrated circuit storage device. The storage device 150 may also be a drive or the like that reads and writes various types of information from and to a portable storage medium such as a flash memory, a CD-ROM, or a DVD. The storage device 150 is not necessarily required to be implemented by a single storage device. For example, the storage device 150 may be implemented by a plurality of storage devices. The storage device 150 may also be replaced by a cloud storage.

The storage device 150 may store one or more programs for executing an optical measurement method according to the present disclosure. The program may be stored in advance in the storage device 150, for example, or may be stored in a non-transitory storage medium, distributed, and read out from the non-transitory storage medium to be installed in the storage device 150, for example. The program may also be downloaded from a network to be installed in the storage device 150, for example.

The optical measurement device 110 includes a substrate 111, a reaction tank 112, a magnetic field application unit 113, and a light detection unit 114. The signal processing device 120 includes a magnetic field control unit 121, an optical control unit 122, an inspection value acquisition unit 123, a determination unit 124, and a communication unit 125.

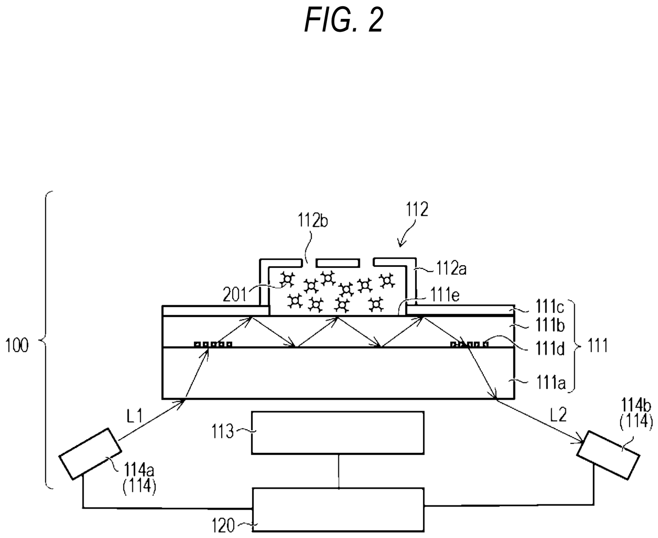

An example of a specific configuration of the optical measurement device 110 and the signal processing device 120 among the respective devices that form the optical measurement system 100 is schematically illustrated in Fig. 2.

As illustrated in Fig. 2, the substrate 111 includes a base portion 111a, an optical waveguide 111b provided on the base portion 111a, a protective film 111c that covers a part of a surface of the optical waveguide 111b, and gratings 111d provided at an interface between the base portion 111a and the optical waveguide 111b.

The reaction tank 112 is provided on the substrate 111 and has, at a bottom portion, a detection surface 111e formed by a part of the surface of the optical waveguide 111b. A frame 112a that forms a part of an outer casing of the reaction tank 112 is formed on the protective film 111c so as to surround the detection surface 111e. The frame 112a has a drip hole 112b formed therein, and the drip hole 112b is communicated with an inside of the reaction tank 112 through a flow path. The reaction tank 112 further includes a lid (not shown) configured to be able to cover the drip hole 112b. The frame 112a is made of, for example, a resin such as acrylonitrile butadiene styrene (ABS), and may be colored black for a light-shielding purpose.

The magnetic field application unit 113 is configured to apply a magnetic field to magnetic particles 201 dispersed in a sample accommodated in the reaction tank 112 and generate a magnetic force on the magnetic particles 201 in a direction toward the detection surface 111e. In this embodiment, the magnetic field application unit 113 is provided at a position facing the base portion 111a such that the substrate 111 is interposed between the magnetic field application unit 113 and the reaction tank 112.

The light detection unit 114 is a functional unit that performs optical detection, and includes a light source 114a and a light detector 114b. The light source 114a irradiates the substrate 111 (base portion 111a) with light L1 to cause light to enter the optical waveguide 111b, and the light detector 114b detects light L2 emitted from the substrate 111 after being reflected by the detection surface 111e. The light detector 114b also generates a light detection signal representing an intensity of the detected light L2.

In this case, two gratings 111d are provided before and after the detection surface 111e in a propagation direction of light in the optical waveguide 111b. Of the two gratings 111d, one is for causing light to enter the optical waveguide 111b, and the other is for emitting light from the optical waveguide 111b. That is, the gratings 111d have a structure that reflects (diffracts) light, and are configured such that light emitted from the light source 114a and caused to enter the optical waveguide 111b is emitted from the optical waveguide 111b after undergoing reflection by the detection surface 111e.

The light source 114a, the gratings 111d, and the optical waveguide 111b form an optical system for light detection in the optical measurement system 100, and the optical system may further include an optical component such as an additionally provided lens.

The signal processing device 120 is connected to each of the magnetic field application unit 113, the light source 114a, and the light detector 114b so as to enable communication therebetween.

The base portion 111a is a member having optical transparency, and is made of, for example, alkali-free glass.

As the optical waveguide 111b, for example, a planar optical waveguide can be used. For example, the optical waveguide 111b can be formed of a thermosetting resin such as a phenol resin, an epoxy resin, or an acrylic resin, or can be formed of a photocurable resin or alkali-free glass. In addition, the optical waveguide 111b is transmissive to predetermined light, and is preferred to be made of, for example, a resin having a refractive index higher than that of the base portion 111a. A part of the surface of the optical waveguide 111b forms the detection surface 111e (sensing area), and the detection surface 111e (sensing area) means a region in which near-field light (evanescent light) that occurs on the surface of the optical waveguide 111b can occur.

The protective film 111c is, for example, a resin film having a low refractive index.

The magnetic field application unit 113 can include, for example, a permanent magnet or an electromagnet. For example, when an electromagnet is used, the magnetic field application unit 113 can maintain a specific magnetic field state by including a processing circuit having a magnetic field control function.

Examples of the light source 114a include a red laser diode, but a laser diode of another color may be used, or a light-emitting diode may be used. The light L1 emitted from the light source 114a may be shaped to be substantially parallel by an additionally provided lens or the like. Examples of the light detector 114b include a photodiode.

The signal processing device 120 is a processor that functions as a control center of the optical measurement system 100. The signal processing device 120 executes programs stored in the storage device 150 or the like, to thereby implement functions corresponding to the programs, namely, respective functions of the magnetic field control unit 121, the optical control unit 122, the inspection value acquisition unit 123, the determination unit 124, and the communication unit 125.

In this embodiment, an example in which the above-mentioned respective functions are implemented by a single physical processor is described, but the present disclosure is not limited thereto. For example, the signal processing device 120 may be configured by combining a plurality of independent processors, and the above-mentioned respective functions may be implemented by the respective processors executing programs.

The signal processing device 120 may be a computer (or a CPU, a micro controller unit (MPU), or the like), or may be a device including, for example, a circuit (such as an ASIC) that implements one or more functions. The signal processing device 120 can also include an amplifier, an A/D converter, a field programmable gate array (FPGA) chip, and the like for performing amplification processing and digital conversion processing on an analog electric signal output by the light detector 114b through a function of the inspection value acquisition unit 123. Details of the above-mentioned functions included in the signal processing device 120 are described later.

Next, the magnetic particles 201 that are used in the optical measurement method according to the present disclosure are described.

The magnetic particles 201 can behave as an accumulation of magnetic material by forming an aggregate through a target substance in the reaction tank 112. As a result, the aggregate formed through the target substance can have increased responsiveness to a magnetic field as compared to individual magnetic particles.

A structure of the magnetic particle 201 is not particularly limited, and the magnetic particle 201 having a suitable structure can be used as appropriate in accordance with a target substance to be measured, a sample containing the target substance, or a purpose and a measurement environment of an apparatus or the like to be used. Examples of the structure of the magnetic particle 201 include a structure in which magnetic nanoparticles are dispersed in a resin or silica particle serving as a core, a structure in which magnetic nanoparticles are immobilized as a shell structure on a surface layer of a core particle, and a structure in which magnetic nanoparticles form secondary particles in a clustered manner.

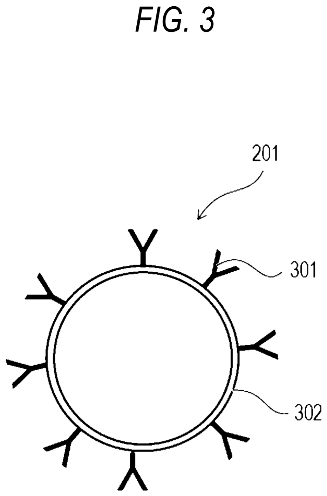

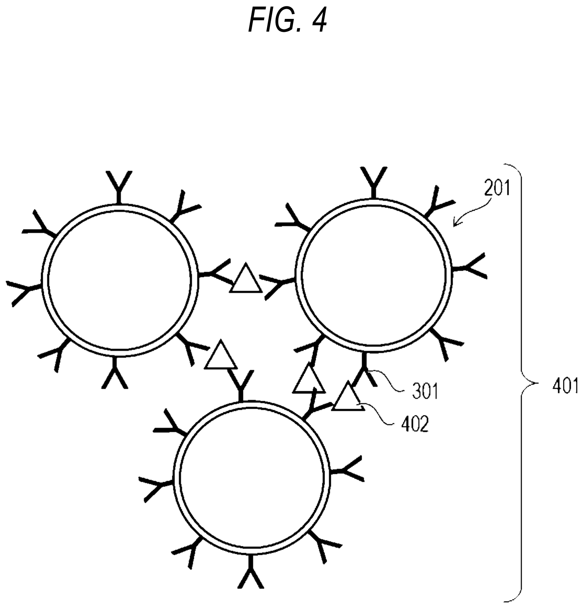

Fig. 3 is a cross-sectional view for illustrating the structure of the magnetic particle 201 on which a substance that specifically binds to a target substance is immobilized. On a surface of the magnetic particle 201, a substance 301 that specifically binds to the target substance is immobilized. Further, on an outermost surface of the magnetic particle 201 illustrated in Fig. 3, a hydrophilic layer 302 formed of a resin is formed in order to suppress non-specific adsorption caused by proteins in a specimen. The substance 301 that specifically binds to the target substance in the specimen is bound to the hydrophilic layer 302 on the outermost surface of the magnetic particle 201, and is configured as illustrated in Fig. 4 such that, when each substance 301 captures one or more target substances 402, the magnetic particles 201 form an aggregate 401 with each other through the target substance. Examples of a mechanism for agglomerating the magnetic particles 201 to form the aggregate 401 can include, when the target substance 402 is an antigen, selecting an antibody as the substance 301 that specifically binds to the target substance 402 and forming an immune complex in which the target substance 402 is sandwiched by the antibodies. In addition, in order to suppress non-specific adsorption between the magnetic particles 201, any hydrophilic polymer may be further immobilized on the surface of each magnetic particle 201 in addition to the substance 301 that specifically binds to the target substance 402.

A combination of the target substance 402 and the substance 301 that specifically binds to the target substance 402 is not limited to the above-mentioned combination of an antigen and an antibody. Examples of other combinations include a combination of a sugar and a lectin, a combination of a nucleotide chain and a nucleotide chain that is complementary thereto, and a combination of a ligand and a receptor.

The magnetic particles 201 are not particularly limited, and one that generally used for detecting the target substance 402 can be selected as appropriate in accordance with a purpose and can also be used in the optical measurement method according to the present disclosure. A particle diameter and a specific gravity of the magnetic particles 201 are also not particularly limited, but the specific gravity of the magnetic particles 201 is preferred to be greater than a specific gravity of a sample in which the magnetic particles 201 are dispersed. Specifically, for example, the particle diameter can be set to 0.1 µm or more and 3.0 µm or less, and the specific gravity can be set to about 1.05 or more and about 3.00 or less. A relationship between the particle diameter and the specific gravity of the magnetic particles 201 is important. When the specific gravity is large, the particle diameter may be reduced, and when the specific gravity is small, the particle diameter may be increased. However, even when the specific gravity is small, the magnetic particles 201 having an excessively large particle diameter may be unsuitable. Thus, it is preferred to select the magnetic particles 201 having a suitable particle diameter and specific gravity as appropriate in accordance with, for example, the specific gravity of the sample in which the magnetic particles 201 are dispersed and conditions of an apparatus to be used, such as an intensity of a magnetic field applied by the magnetic field application unit 113.

Next, a procedure for carrying out the optical measurement method according to the present disclosure by the optical measurement system 100 according to this embodiment is described. In the following description, the target substance 402 is assumed to be an antigen, and the substance 301 that specifically binds to the target substance 402 and is immobilized on the magnetic particle 201 is assumed to be a monoclonal antibody, a mixture of two or more monoclonal antibodies, or a polyclonal antibody.

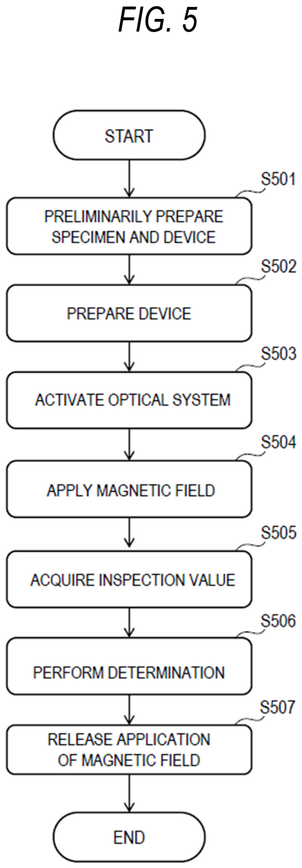

Fig. 5 is a flow chart for illustrating a flow of the optical measurement method carried out by the optical measurement system 100.

First, in a preliminary preparation step of Step S501, a specimen to be measured and the optical measurement device are prepared.

Specifically, as the preliminary preparation step, a specimen that may contain the target substance 402 is collected, and the device is activated. Types of specimens that can be used include nasal discharge, saliva, sputum, blood, lymph, and any other substance that can be collected from a human body. As one example, when the specimen is a nasal swab specimen, the nasal swab specimen is collected by scraping a nasopharynx with a sterilized cotton swab, and the sterilized cotton swab is immersed in and stirred with an extraction buffer in an extraction buffer tube. Thus, the specimen is suspended in the extraction buffer that is an aqueous solution containing a surfactant, and the suspension is introduced into the reaction tank 112 of the optical measurement device 110. The extraction buffer tube refers to a tube made of a resin or glass that contains the extraction buffer.

Subsequently, in a preparation step of Step S502, the optical measurement device 110, the reaction tank 112 in the optical measurement device (110) accommodating a sample containing magnetic particles 201 on each of which the substance 301 that specifically binds to the target substance 402 is prepared.

A sample containing the specimen prepared in Step S501 is introduced into the reaction tank 112, and the sample and the magnetic particles 201 on each of which the substance 301 that specifically binds to the target substance 402 is immobilized are further mixed in the reaction tank 112. Thus, simultaneously with the introduction of the sample, binding of the target substance 402 and the substance 301 that specifically binds to the target substance 402 (antigen-antibody reaction) is initiated.

A specific method of mixing the sample and the magnetic particles 201 in the reaction tank 112 is not particularly limited, but the mixing can be performed as follows.

Examples thereof can include a configuration in which the magnetic particles 201 are immobilized on a filter to be attached to the extraction buffer tube, and when a worker causes the sample to drip into the reaction tank 112, the sample and the magnetic particles 201 are mixed.

As another example, a configuration in which the magnetic particles 201 are held on the detection surface 111e provided at a bottom surface of the reaction tank 112 by a sealing film can be employed. The sealing film is formed of a material substance containing at least a water-soluble substance and allowing, when the sample is introduced into the reaction tank 112, the water-soluble substance to rapidly dissolve and the magnetic particles 201 to be dispersed in the sample. A material substance for the water-soluble substance can be selected from publicly-known excipients, for example, saccharides, starches, celluloses, and inorganic salts.

Subsequently, in an optical-system activation step of Step S503, an optical system in the optical measurement device 110 is activated. In response to reception of a trigger for activating the optical system, the optical measurement device 110 activates the optical system under control of the optical control unit 122 included in the signal processing device 120. The trigger for activating the optical system can be optionally set, and examples thereof can include closing the lid of the reaction tank 112 after the sample has been introduced into the reaction tank 112, and the worker inputting an instruction to activate the optical system from the input device 130 to the communication unit 125 of the signal processing device 120.

When the optical system is activated, the light source 114a emits the light L1, and the light detector 114b detects the light L2 emitted from the substrate 111. The light detection signal representing a light amount (intensity) of the detected light L2 is output to the signal processing device 120. The optical-system activation step of Step S503 can also be performed after initiation of application of a magnetic field in the subsequent magnetic field application step of Step S504.

Subsequently, in the magnetic field application step of Step S504, a magnetic field is applied to the magnetic particles 201 by the magnetic field application unit 113 under control of the magnetic field control unit 121 included in the signal processing device 120. In the optical measurement method according to the present disclosure, during measurement, the sample is held under a state in which a magnetic force is generated on the magnetic particles 201 in the reaction tank 112 in the direction toward the detection surface 111e by the magnetic field application unit 113.

The application of a magnetic field can be initiated at any timing, and can be initiated, for example, at a timing at which the sample has flowed into the reaction tank 112 and the detection surface 111e at the bottom surface is sufficiently covered. As another example, the application of a magnetic field can be initiated after a predetermined time has elapsed for the magnetic particles 201 to capture the target substances 402 in the sample.

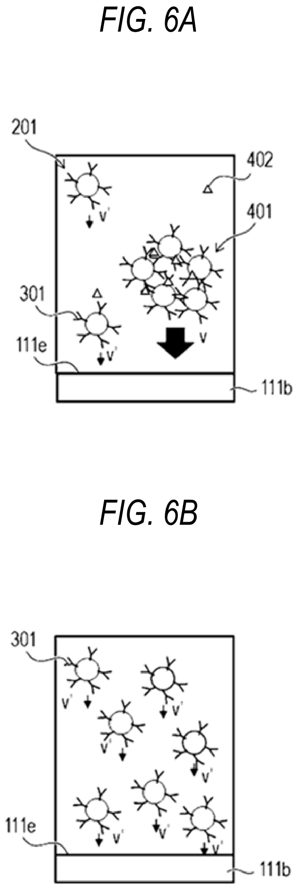

Fig. 6A is a schematic view for illustrating behavior of the magnetic particles 201 exhibited when a magnetic field is applied to a positive specimen containing the target substances 402. Fig. 6B is a schematic view for illustrating behavior of the magnetic particles 201 exhibited when a magnetic field is applied to a negative specimen not containing the target substances 402.

According to Fig. 6A, the reaction tank 112 is filled with the sample, and the magnetic particles 201 and the target substances 402 (antigens) are suspended in the sample. When the antigens are present, agglomeration of the magnetic particles 201 is promoted due to the immune complex formation. Accordingly, when a magnetic field is applied, in the positive specimen, the magnetic particles 201 move under a state in which two or more magnetic particles 201 are bound to each other through the antigens, in the direction toward the detection surface 111e by the magnetic field. A velocity thereof at this time is set as "v."

Meanwhile, according to Fig. 6B, in the negative specimen, the magnetic particles 201 are in a mutually dispersed state, and, when a magnetic field is applied, individually move in the direction toward the detection surface 111e. A velocity thereof at this time is set as "v'."

Comparison between the velocity "v" and the velocity v' at this time results in v>v', and it can be said that the magnetic particles 201 agglomerated by the antigens (aggregate 401) have a faster sedimentation velocity exhibited when a magnetic field is applied than the individual magnetic particles 201. Then, the agglomerated magnetic particles 201 settle and deposit on the detection surface 111e earlier. That is, in this embodiment, when a magnetic field is applied in the direction toward the detection surface 111e to the magnetic particles 201 in the reaction tank 112, the aggregate 401 is preferentially sedimented and settled on the detection surface 111e. Accordingly, detection sensitivity for the target substance 402 can be improved.

In this embodiment, the intensity of the magnetic field applied by the magnetic field application unit 113 may be an intensity that moves the magnetic particles 201 individually, but it is more preferred to select such an intensity of the magnetic field as to substantially move only the agglomerated magnetic particles 201 without moving the individual magnetic particles 201. Through selection of the magnetic field that moves only the agglomerated magnetic particles 201, it is possible to suppress detection signals attributable to the individual magnetic particles 201. That is, through selection of such an intensity of the magnetic field as to selectively move only the agglomerated magnetic particles 201, the agglomerated magnetic particles 201 can be moved more selectively to the detection surface 111e, thereby enabling determination of positive or negative with higher accuracy.

Subsequently, in an inspection value acquisition step of Step S505, the inspection value acquisition unit 123 of the signal processing device 120 acquires an inspection value based on the light amount of the light detected by the light detection unit 114 under a state in which a magnetic field is applied.

In this embodiment, the inspection value is acquired based on the signal intensity of the light detection signal generated by the light detector 114b and transmitted to the signal processing device 120, the light detection signal representing the light amount (intensity) of the light L2.

The inspection value acquired by the inspection value acquisition unit 123 can assume a value corresponding to the light amount of the light detected by the light detection unit 114. For example, the inspection value may be the signal intensity itself of the light detection signal representing the intensity of the light L2 generated by the light detector 114b.

Further, the inspection value may assume a value corresponding to a difference between the light amount of the light L2 detected by the light detection unit 114 and a predetermined reference light amount.

The reference light amount can be, for example, the intensity of the light L2 detected by the light detector 114b when the light is totally reflected in the optical waveguide. Such a reference light amount may be obtained by, for example, measuring the intensity of the light L2 after the reaction tank 112 is filled with water in advance. In another case, the intensity of the light L2 detected by the light detector 114b when the reaction tank 112 is empty may be used as the reference light amount.

The inspection value may also assume a value indicating a degree of change in the light amount from the light amount of the light detected by the light detection unit 114 at a reference time point.

The reference time point can be optionally determined, and specific examples thereof may include a time point at which the detection surface 111e is completely covered with the sample accommodated in the reaction tank 112. For example, a difference serving as the degree of change from the light amount of the light L2 detected by the light detector 114b at the reference time point may be taken, and this difference can be used as the inspection value. The degree of change in the light amount from the light amount of the light detected by the light detection unit 114 at the reference time point is not limited to the difference. For example, the inspection value may be a rate or the like of the change in the light amount of the light detected by the light detection unit 114 at the reference time point.

Further, as the reference time point, a time point at which a predetermined time has elapsed after the detection surface 111e is completely covered with the sample may be used.

The signal intensity of the light detection signal transmitted from the light detector 114b to the signal processing device 120, the light detection signal representing the light amount (intensity) of the light L2, and the inspection value acquired based on the signal intensity can be stored in the storage device 150.

The storage device 150 can record information regarding defective light detection signals in, for example, a case in which introduction of the sample into the reaction tank 112 is improper or a case in which the sample contains an interfering substance or an abnormal specimen. The inspection value acquisition unit 123 can be configured to output a measurement error when the corresponding information applies.

Subsequently, in a determination step of Step S506, the determination unit 124 of the signal processing device 120 determines presence or absence of the target substance 402 in the sample based on the inspection value acquired by the inspection value acquisition unit 123.

In the optical measurement method according to the present disclosure, the difference in the velocity at which the magnetic particles 201 agglomerated through the target substance 402 and the individually dispersed magnetic particles 201 move toward the detection surface 111e is utilized as described above. That is, when the sample contains the target substances 402, the magnetic particles 201 settle on the detection surface 111e earlier than when the sample does not contain the target substances 402. Thus, the presence or absence of the target substance 402 in the sample is determined based on the fact that a decrease in the signal intensity of the light detection signal detected by the light detector 114b is observed in a shorter time after the initiation of the application of a magnetic field. The optical measurement method according to the present disclosure does not require a step of applying a magnetic field for generating a magnetic force in a direction away from the detection surface 111e to separate the magnetic particles 201 from the detection surface 111e, the step being essential in the optical measurement method according to the related art. As a result, the determination of the presence or absence of the target substance 402 using the magnetic particles 201 can be achieved in a shorter time.

Further, in the optical measurement method according to the present disclosure, unlike the related art, the magnetic particles 201 that are not bound to the target substances 402 are not separated from magnetic particles 201 bound to the detection surface 111e through the target substance 402 by causing a magnetic force to pull the magnetic particles 201 away from the detection surface 111e. Thus, in the optical measurement device used in the optical measurement method according to the present disclosure, a substance that binds to the target substance 402 is not required to be immobilized on the detection surface 111e. Accordingly, a simple and low-cost device can be used for the measurement. In the present disclosure, "a substance that specifically binds to the target substance is not immobilized on the detection surface" indicates that the detection surface originally not having the substance has not been treated to immobilize the substance.

A specific example of the determination performed by the determination unit 124 is further described below.

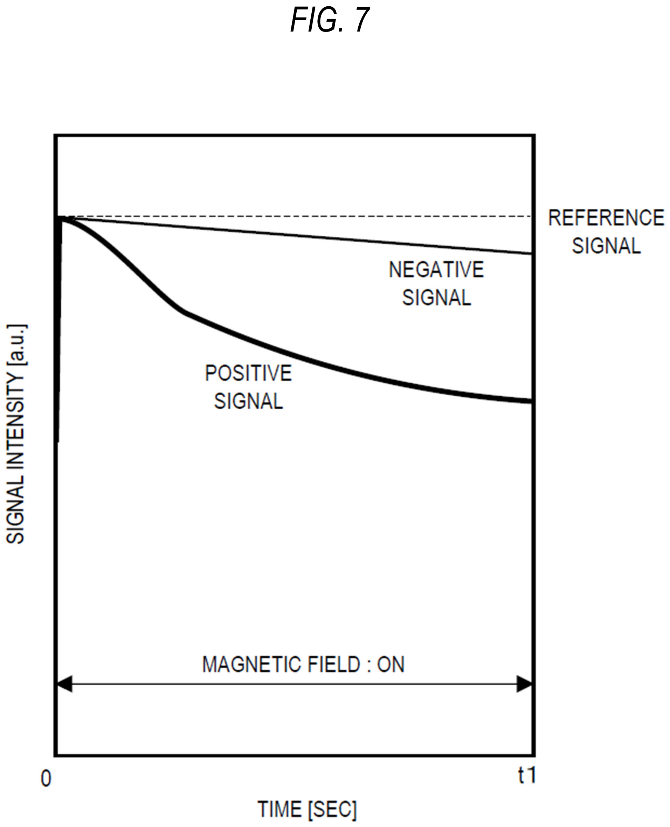

Fig. 7 is a graph showing an example of temporal changes in the signal intensity of the light detection signal representing the intensity of the light L2 detected by the light detection unit 114.

The graph of Fig. 7 has a vertical axis indicating the signal intensity [a.u.] of the light detection signal representing the intensity of the light L2 generated by the light detector 114b, and a horizontal axis indicating time [sec]. The thick line in the graph of Fig. 7 indicates a temporal change in the signal intensity of a light detection signal (positive signal) obtained for a sample containing target substances, and the thin line in the graph indicates a temporal change in the signal intensity of a light detection signal (negative signal) obtained for a sample not containing the target substances.

The graph shown in Fig. 7 is an example of a case in which the introduction of the sample into the reaction tank 112 and the initiation of the application of a magnetic field were performed at almost the same time, and the temporal changes in the signal intensity were obtained with this time point set as 0.

In a case in which the sample is not accommodated in the reaction tank 112 and the reaction tank 112 is in an empty state, when the light L1 is emitted from the light source 114a, the light propagating through the optical waveguide 111b is not totally reflected by the detection surface 111e, and the light detection signal has a lower value than in a case of total reflection.

When the sample reaches the detection surface 111e and the detection surface 111e is covered with the sample, the light propagating through the optical waveguide 111b is totally reflected by the detection surface 111e, and the light detection signal increases. Thus, in Fig. 7, light signal intensities of both the positive signal and the negative signal rise sharply at the time 0.

In Fig. 7, a time point at which the detection surface 111e was completely covered with the sample and the light amount of the light L2 detected by the light detector 114b reached a maximum value is set as the reference time point, and the signal intensity of the light detection signal at this reference time point is shown as a reference signal.

When the magnetic particles 201 settle on the detection surface 111e, a ratio at which the light propagating through the optical waveguide 111b is totally reflected by the detection surface 111e decreases, and the light signal intensity generated by the light detector 114b also decreases. That is, in this embodiment, the number of the magnetic particles 201 in the sample that settle on the detection surface 111e due to the magnetic field increases over time, and hence the intensity of the light L2 detected by the light detector 114b tends to decrease over time.

At this time, as described above, in the positive specimen, the magnetic particles 201 are agglomerated through the target substance 402, resulting in a greater movement velocity toward the detection surface 111e. Thus, the positive signal exhibits a greater temporal change in the detection intensity than the negative signal. Accordingly, it is possible to discriminate the positive specimen and the negative specimen as shown in Fig. 7.

For example, in the example shown in Fig. 7, when the signal intensity itself of the light detection signal representing the intensity of the light L2 generated by the light detector 114b is used as the inspection value, the positive specimen and the negative specimen can be discriminated from a degree of temporal decrease in the inspection value.

Further, for example, in the example shown in Fig. 7, when the difference in the signal intensity between a value of the positive signal or the negative signal and the reference signal is used as the inspection value, the inspection value changes in an increasing direction as the intensity of the light L2 detected by the light detection unit 114 decreases, that is, in a direction opposite to that of the intensity of the light L2. Accordingly, in this case, the positive specimen and the negative specimen can be discriminated from a degree of temporal increase in the inspection value.

In the discrimination between the positive specimen and the negative specimen based on the inspection value, a predetermined threshold value can be used. That is, the determination step of Step S506 can include determining the presence or absence of the target substance 402 in the sample based on the inspection value acquired in Step S505 and the predetermined threshold value.

For example, in the example shown in Fig. 7, a case in which a point in time at which the signal intensity of the light detection signal has decreased to 60% of the signal intensity of the reference signal is used as a basis for threshold value setting is considered. At this time, for example, when the signal intensity of the light detection signal representing the intensity of the light L2 generated by the light detector 114b is used as the inspection value, the value corresponding to 60% of the signal intensity of the reference signal can simply be used as the threshold value. Further, when the rate (%) of change from the signal intensity of the reference signal is used as the inspection value, 40(%) can be set as the threshold value.

As another form, the determination step can include acquiring temporal transition information (hereinafter referred to as "transition information") regarding the inspection value, and determining positive or negative based on a moving average value or an integrated value of the inspection value during a specific period of time and a predetermined threshold value.

The transition information can be obtained by, for example, acquiring information regarding a temporal change in the light detection signal from the light detector 114b and deriving the transition information based on the acquired information. An example of acquiring the information regarding the temporal change in the light detection signal from the light detector 114b can include acquiring the signal intensity of the light detection signal while updating a minimum value of the signal intensity of the light detection signal at a certain time point after magnetic field application (immediately after the magnetic field application or after a lapse of a predetermined time therefrom).

The threshold value can be stored in advance in the storage device 150.

The determination step may include performing the determination through use of reference information relating to a relationship between an elapsed time from the reference time point and the inspection value.

In this case, the reference information may be the transition information obtained in advance through use of a sample having a known concentration of the target substances 402. That is, for example, the reference information can include information relating to an inspection value regarding a sample (negative specimen) having a concentration of the target substances 402 less than a predetermined value. Further, for example, the reference information can include information relating to an inspection value regarding a sample

(positive specimen) having a concentration of the target substances 402 equal to or greater than the predetermined value.

Further, for example, the reference information may be transition information regarding a sample containing the target substance 402 at a specific concentration, the transition information being predicted and obtained through computer simulation.

The reference information may be stored in advance in the storage device 150 as a look-up table (LUT) or a mathematical expression.

The determination unit 124 can perform the determination based on a correspondence relationship between the inspection value and the reference information.

Examples of performing the determination through use of the reference information obtained based on the positive specimen include acquiring the transition information through use of a sample containing the target substance 402 at a limit of detection and using the acquired transition information for the determination. For example, a value of the inspection value obtained for the positive specimen after a lapse of a predetermined time from the application of a magnetic field is set as a threshold value, and the determination of positive or negative can be output when an inspection value obtained for an actual sample exceeds (or falls below) the threshold value. Further, a moving-average value or an integrated value of the inspection value obtained for a positive specimen during a period from the application of a magnetic field until a lapse of a predetermined time can also be set as the threshold value.

The reference information can also include information relating to the inspection value for each of a plurality of samples having mutually different concentrations of the target substances 402. In this case, the determination unit 124 not only can determine positive or negative but also can estimate a concentration range of the target substances 402 contained in the sample by performing comparison between the transition information for the plurality of positive specimens having different concentrations and the inspection value (transition information) obtained for an actual sample.

Further, as another example, the determination can be performed through regression analysis based on the transition information regarding a plurality of positive specimens having different concentrations. Specifically, a cross-correlation coefficient shown below can be used to perform the determination of positive or negative and the estimation of the concentration of the target substances 402 based on a degree of similarity between the transition information obtained for a positive specimen and the transition information obtained for an actual sample. $r \left(xy\right) = \frac{{\sum }_{i = 1}^{N} \left({x}_{i}-\overline{x}\right) \left({y}_{i}-\overline{y}\right)}{\sqrt{{\sum }_{i = 1}^{N} {\left({x}_{i}-\overline{x}\right)}^{2}} ⋅ \sqrt{{\sum }_{i = 1}^{N} {\left({y}_{i}-\overline{y}\right)}^{2}}} ,$ where $\overline{x} = \frac{1}{N} {\sum }_{i = 1}^{N} {x}_{i} \overline{y} = \frac{1}{N} {\sum }_{i = 1}^{N} {y}_{i} .$
X: transition information obtained for an actual sample
Y: transition information regarding a positive specimen obtained from the LUT

Examples of performing the determination from the reference information obtained based on the negative specimen include acquiring the transition information through use of a sample containing the target substance 402 at a concentration lower than the limit of detection and using the acquired transition information for the determination. For example, a threshold value is set based on a value of the inspection value obtained for the negative specimen after a lapse of a predetermined time from the application of a magnetic field, and the determination of positive or negative can be output when an inspection value that exceeds (or falls below) the threshold value has been obtained. Further, a moving-average value or an integrated value of the inspection value obtained for a negative specimen during a period from the application of a magnetic field until a lapse of a predetermined time can also be set as the threshold value.

As another example, the determination unit 124 can output a negative determination when a degree of similarity between the transition information obtained for a negative specimen through use of the above-mentioned cross-correlation coefficient and the transition information obtained for an actual sample is high, and can output a positive determination when the degree of similarity is low. When the determination is performed through use of the cross-correlation coefficient, for example, a threshold value (for example, a cross-correlation coefficient r>0.9) stored in advance in the storage device 150 can be used for the determination.

After the determination of positive or negative is performed in Step S506, the application of a magnetic field is released in a magnetic field application release step of Step S507, and the measurement is terminated. A result of the determination output by the determination unit 124 is sent to the output device 140 by the communication unit 125, and the worker is notified of the result of the determination.

A test kit according to the present disclosure is a test kit for use in the optical measurement method described above, and includes the magnetic particle 201 on which a substance that specifically binds to the target substance 402 is immobilized, the substrate 111 having the optical waveguide 111b, and the reaction tank 112. As described above, the reaction tank 112 is provided on the substrate 111, and has, at the bottom portion, the detection surface 111e, which is formed by a part of the surface of the optical waveguide 111b, and on which a substance that specifically binds to the target substance 402 is not immobilized.

Any one of the embodiments described above merely indicates an example of implementation of the present disclosure, and the technical scope of the present disclosure is not to be construed in a limiting manner due to those embodiments. That is, the present disclosure can be carried out in various forms without departing from the technical spirit of the present disclosure or major features of the present disclosure. For example, an embodiment in which a configuration of a part of any one of the embodiments is added to another embodiment or an embodiment in which a configuration of a part of any one of the embodiments is substituted by a configuration of a part of another embodiment is also to be understood as an embodiment of the present disclosure.

Various embodiments have been described in detail above but it will be understood that the present disclosure is not limited to these embodiments and encompasses all modifications, variants, alternatives and equivalents falling within the scope of the appended claims.

## Claims

1. An optical measurement method using an optical measurement device (110),
the optical measurement device (110) including:
a substrate (111) including an optical waveguide;
a reaction tank (112), which is provided on the substrate (111), and has, at a bottom portion, a detection surface formed by a part of a surface of the optical waveguide;
a magnetic field application unit (113) configured to apply a magnetic field to magnetic particles dispersed in a sample accommodated in the reaction tank (112) and generate a magnetic force on the magnetic particles in a direction toward the detection surface; and
a light detection unit (114) configured to cause light to enter the optical waveguide, and detect light emitted from the substrate (111) after being reflected by the detection surface,
the optical measurement method comprising:
a preparation step of preparing the optical measurement device (110), the reaction tank (112) in the optical measurement device (110) accommodating the sample containing the magnetic particles on each of which a substance that specifically binds to a target substance is immobilized;
a magnetic field application step of applying the magnetic field to the magnetic particles by the magnetic field application unit (113);
an inspection value acquisition step of acquiring an inspection value based on an amount of light detected by the light detection unit (114) under a state in which the magnetic field is applied; and
a determination step of performing determination of one of presence or absence of the target substance in the sample based on the inspection value.

2. The optical measurement method according to claim 1, wherein the inspection value assumes a value corresponding to the light amount of the light detected by the light detection unit (114).

3. The optical measurement method according to claim 1, wherein the inspection value assumes a value corresponding to a difference between the light amount of the light detected by the light detection unit (114) and a predetermined reference light amount.

4. The optical measurement method according to claim 1, wherein the inspection value assumes a value indicating a degree of change in the light amount from the light amount of the light detected by the light detection unit (114) at a reference time point.

5. The optical measurement method according to any one of claims 1 to 4, wherein a substance that specifically binds to the target substance is not immobilized on the detection surface.

6. The optical measurement method according to any one of claims 1 to 5, wherein the determination step includes performing the determination through use of reference information relating to a relationship between an elapsed time from a reference time point and the inspection value.

7. The optical measurement method according to claim 6, wherein the reference information includes information relating to the inspection value regarding the sample having a concentration of the target substance less than a predetermined value.

8. The optical measurement method according to claim 6, wherein the reference information includes information relating to the inspection value regarding the sample having a concentration of the target substance equal to or greater than a predetermined value.

9. The optical measurement method according to any one of claims 1 to 8, wherein the determination step includes performing the determination based on the inspection value and a predetermined threshold value.

10. The optical measurement method according to any one of claims 1 to 8, wherein the determination step includes performing the determination based on a moving average value of the inspection value and a predetermined threshold value.

11. The optical measurement method according to any one of claims 1 to 8, wherein the determination step includes performing the determination based on an integrated value of the inspection value and a predetermined threshold value.

12. The optical measurement method according to claim 8, wherein the reference information includes information relating to the inspection value regarding each of a plurality of the samples having mutually different concentrations of the target substances.

13. The optical measurement method according to any one of claims 1 to 12, wherein the magnetic particles comprise particles that have a property to be agglomerated through the target substance.

14. The optical measurement method according to claim 13, wherein the magnetic field application step includes applying such a magnetic field intensity as to selectively move the agglomerated magnetic particles.

15. An optical measurement system (100) comprising:
a substrate (111) including an optical waveguide;
a reaction tank (112), which is provided on the substrate (111), and has, at a bottom portion, a detection surface formed by a part of a surface of the optical waveguide;
a magnetic field application unit (113) configured to apply a magnetic field to magnetic particles, which are dispersed in a sample accommodated in the reaction tank (112), and on each of which a substance that specifically binds to a target substance is immobilized, and generate a magnetic force on the magnetic particles in a direction toward the detection surface;
a light detection unit (114) configured to cause light to enter the optical waveguide, and detect light emitted from the substrate (111) after being reflected by the detection surface;
an inspection value acquisition unit (123) configured to acquire an inspection value based on an amount of light detected by the light detection unit (114) under a state in which the magnetic field is applied; and
a determination unit (124) configured to determine one of presence or absence of the target substance in the sample based on the inspection value.

16. The optical measurement system (100) according to claim 15, wherein a substance that specifically binds to the target substance is not immobilized on the detection surface.

17. A test kit for use in the optical measurement method of any one of claims 1 to 14, the test kit comprising:
a magnetic particle on which a substance that specifically binds to a target substance is immobilized;
a substrate (111) including an optical waveguide; and
a reaction tank (112), which is provided on the substrate (111), and has, at a bottom portion, a detection surface, which is formed by a part of a surface of the optical waveguide, and on which a substance that specifically binds to the target substance is not immobilized.
